# EUROPEAN PATENT SPECIFICATION

(11) **EP 1 879 564 B1**
(45) Date of publication and mention of the grant of the patent: **23.12.2009**
(21) Application number: 06740648.8
(22) Date of filing: 07.04.2006
(51) Int. Cl.: A61K 31/166, A61K 31/4412, C07C 237/30, C07D 213/74, C07D 223/22, C07D 241/44, C07D 215/48, C07D 209/42, C07D 471/04, C07D 233/70, C07D 235/26, C07D 263/38, C07D 263/58, C07D 213/63, A61P 3/10

(54) **COMPOUNDS TO TREAT AMYLOIDOSIS AND PREVENT DEATH OF BETA-CELLS IN TYPE 2 DIABETES MELLITUS**
VERBINDUNGEN ZUR BEHANDLUNG VON AMYLOIDOSE UND VERHINDERUNG DES TODS VON BETA-ZELLEN BEI DIABETES MELLITUS TYP 2
COMPOSES UTILES POUR TRAITER L'AMYLOSE ET PREVENIR LA MORT DES CELLULES BETA DANS LE DIABETE SUCRE DE TYPE 2

(30) Priority: 07.04.2005 US 669411 P
(43) Date of publication of application: 23.01.2008
(73) Proprietor: Astrum Therapeutics Pty, Ltd., Melbourne VIC 3000 (AU)
(72) Inventor: ZOLOTOY, Alexander, Richmond, British Columbia V6Y 1K2 (CA); HAYES, Eric, Seattle, Washington 98117 (US)
(74) Representative: Grünecker, Kinkeldey, Stockmair & Schwanhäusser Anwaltssozietät
(86) International application number: PCT/US2006/012875
(87) International publication number: WO 2006/110477

(56) References cited:
- EP-A- 1 060 750
- WO-A-00/71101
- WO-A-01/49281
- WO-A-02/24652
- WO-A-98/27972
- WO-A-2005/027901

## Description

The present invention discloses compounds and use thereof for the preparation of a medicament to treat patients with type 2 diabetes mellitus (T2DM). The administration of these compounds results In inhibition of amyloidosis and prevention of death of pancreatic β-cells.

Islet cell amyloidosis (IA) is a basic characteristic of the pathology T2DM that is associated with the death of pancreatic β-cells (Kahn et al. Diabetes 1999, 48:241-53; Hopener et al. Mol. Cell Endocrinol. 2002, 197:205-212; O'Brien, Mol Cell Endocrinol. 2002, 197:213-219). As a consequence, β-cell mediated insulin secretion is reduced, aggravating the hyperglycemic diabetic state (Hopener *et al.* 2002, *supra).* Drugs currently available on the market do not prevent IA. A recent study In the UK analyzed the effects (11 year follow-up) of oral glycemic control agents on β-cell function and concluded that IA deposition is not diminished, and may possibly even be aggravated, and that patient β-cell function deteriorates irrespective of treatment (Tumer, Diabetes Care 1998, 21:C35-C38). Thus there is a clear need for new anti-IA therapy.

The deposition of islet amyloid IA within β-cells of the pancreas is one of the main characteristics of T2DM pathology with an incidence of up to 96% (Westermark, lnt J Exp Clin Invest. 1994, 1:47-60). IA has been described In humans, non-human primates and cats but is not found in rat, mouse, rabbit, hamster, hare or dog (Hopener *et al.* 2002, *supra*). In 1987 the structure of the main component of IA was determined Independently by Westermark and Cooper and designated Islet Amyloid Polypeptide (IAPP) or Amylin (Cooper et al. Proc Natl Acad Sci. 1987, 84:8628-8632; Westermark et al. Proc Natl Acad Sci. 1987, 84:3881-3885). It is believed that IAPP, along with insulin and glucagon, is an active islet hormone involved in the metabolic control of glucose metabolism. IAPP is co-secreted with insulin from β-cells of the pancreas. Transformation of IAPP monomers from the alpha-helix (α-helix) to beta-sheet (β-sheet) conformation results in the formation of toxic IAPP fibrils, death of β-cells and subsequent accumulation of IA (Hopener *et al.* 2002_{;} *supra*). Thus the transformation of IAPP impairs insulin release and aggravates the pathology of diabetes.

Reported results of studies support the relationship between IAPP, IA and β-cell death. First, in separate studies 13 of 15 (87%), 20 of 26 (77%) and 22 of 24 (92%) T2DM patients exhibited pancreatic IA compared to 0 of 10 (0%) and 1 of 14 (7%) in controls (Gebre-Medhin et al. Diabetologia 2000, 43:687-695; Clark et al. Lancet 1987, 2:231-234; Clark et al. Diabetologia 1990, 33:285-289). In human diabetics with cystic fibrosis (CF), IA incidence was reported to be 69%, compared to 17% and 0% in borderline T2DM patients and non-diabetic controls, respectively (lannucci et al. Hum Pathol. 1984, 15:278-284). IA occupies up to 80% of islets in T2DM patients (Clark et al. Diabetes Res Clin Pract. 1995, 28:S39-S47). The density of pancreatic β-cells is decreased by 24% (P < 0.05) while α-cell density increased by 58% (P < 0.001) in T2DM subjects compared to controls (Gebre-Medhin *et al.* 2000, *supra).* Modest differences have also been reported in the incidence of IA in T2DM (100%) compared to control (60%) subjects (Westermark et al. Diabetologia 1978, 15:417-421). However, the volume of islets completely free from amyloid in diabetic subjects was 0.41 ± 0.03 cm³ compared to 1.58 ± 0.16 cm³ in non-diabetics subjects (P < 0.05).

Second, in humans the presence of IAPP-induced IA is associated with the loss of 24% to 50% of pancreatic β-cells (Clark et al. Diabetes Res. 1988, 9:151-159; Couce et al. J Clin Endocrinol Metab. 1996, 81:1267-1272). This conclusion was confirmed with respect to differentiation between obese and lean human subjects (Butler et al. Diabetes 2003, 52:2304-2314). Obese subjects with T2DM exhibited a 63% deficit in relative β-cell volume compared to non-diabetic obese subjects (P < 0.01), whereas lean subjects with T2DM exhibited a 41 % decrease in relative β-cell volume compared to non-diabetic lean controls (P < 0.05). The observed decreased β-cell volume in patients with T2DM was due to a specific decrease in the number of β-cells rather than a generalized decrease of total cell volume. The frequency of apoptotic (cell-death) events (frequency of β-cell apoptosis/relative volume of β-cells) was 3 times higher in obese subjects with T2DM compared to obese controls (P < 0.05) and 10 times higher in lean T2DM subjects compared to lean controls (P < 0.05). IA has been observed in 81 % of obese T2DM cases compared to 10% in obese controls (P < 0.01) and in 88% of lean T2DM cases compared to 13% of lean controls (P < 0.01). The frequency and extent of Congo red birefringence of islets (visual measure of IAPP fibril formation and IA deposition) was also significantly higher in obese and lean T2DM patients compared to appropriate controls.

Finally, similar links between IAPP, IA and β-cell death have been demonstrated in various animal models of T2DM. In cats the presence of IA is associated with the loss of up to 50% of β-cells (O'Brien et al. J Comp Pathol. 1986, 96:357-359). Obese non-transgenic mice do not develop diabetes. They adapt to insulin resistance through a 9-fold increase (P < 0.001) in β-cell mass that results from a 1.7-fold increase in islet neogenesis (P < 0.05) and a 5-fold increase in β-cell replication per islet (P < 0.001) compared to non-obese controls. Obese transgenic mice expressing the human IAPP (hIAPP) gene develop midlife diabetes with islet amyloid and an 80% (P < 0.001) decrease in β-cell mass that is not compensated for. The mechanism subserving the failed expansion was a 10-fold increase in β-cell apoptosis compared to controls (P < 0.001). The frequency of β-cell apoptosis correlates with the rate of increase of IAPP fibril formation and IA, but not to the extent of islet amyloid or the blood glucose concentration (Butler *et al.* 2003, *supra*). Additional studies, with hIAPP transgenic mice have demonstrated that amyloid severity (amyloid area/islet area) inversely correlates with viable β-cell densities (r = -0.59, P < 0.0001) (Wang et al. Diabetes 2001, 50:2514-2520).

The development of IA correlates with the development of T2DM. Studies with *Macaca nigra,* a species of old world monkey that develops spontaneous IA and T2DM, have shown that initially IA reduces insulin secretion associated with mild impairments of glucose tolerance without changes in fasting glucose concentrations. Long term studies in the same species indicated continued IA associated with a further reduced insulin secretion profile and deterioration of glucose tolerance. The development of fasting hyperglycemia was a late occurring phenomenon and appeared in animals with substantial IA (Howard CF, Jr. Diabetologia 1986, 29:301-306). *Macaca mulatta* were followed during an entire life span and post-mortem pancreatic tissue from 26 monkeys were examined (de Koning et al. Diabetologia 1993, 36:378-384). Four groups of animals were studied: group I, young (< 10 years), lean and normoglycemic; group II, older (> 10 years), lean or obese, normoglycemic; group III, normoglycemic and hyperinsulinemic; and group IV, diabetic, Islet sizes were larger in animals from groups III (P < 0.01) and IV (P < 0.0001) compared to groups I and II. Amyloid was absent in group I (0%), but small deposits were present in 3 of 9 group II animals (33%) and in 4 of 6 group III animals (75%) and occupied between 0.03% and 45% of the islet area. Amyloid was present in 8 of 8 group IV animals (100%) and occupied between 37% and 81% of islet area. Every islet was affected in 7 of 8 diabetic monkeys (88%). It was concluded that islet amyloid appears to precede the development of overt diabetes in *Macaca mulatta* and is likely to be a factor in the destruction of islet cells and onset of hyperglycemia. IA has also been demonstrated in 79% of diabetic cats, 44% of cats with impaired glucose tolerance and 25% of normal cats (Johnson KH et al. Am. J. Pathol. 1989, 135:245-250). IAPP immunoreactivity was very low in 8 of 8 diabetic cats, was increased in 6 of 6 cats with impaired glucose tolerance and was highest in normal cats. The investigators concluded that the presence of IA and the disappearance of IAPP from β-cell loss predicted impaired glucose tolerance with a probability of 88%.

Transformation of IAPP from the α-helix conformation into the β-sheet conformation results in the formation of toxic IAPP fibrils, IA and the death of pancreatic β-cells. At a concentration of 5 µM and higher, IAPP is stabilized in the fibril form and induces beta-cell death; whereas at 1 µM and lower, IAPP is not in a fibrillogenic form and does not induce beta-cell death. Researchers noted the following manifestations of cytotoxicity: plasma membrane blebbing, inappropriate chromatin condensation and DNA fragmentation (Lorenzo et al. Nature. 1994, 368: 756-760). Recent studies that employed better protein production and stabilization procedures have found the EC₅₀ for IAPP-mediated β-cell cytotoxicity to be approximately 100 nM and confirmed that the cytotoxic form was the fibrillar form of the peptide (Krampert et al. Chem Biol. 2000, 7:855-71). Application of fibrillogenic human IAPP to pure planar lipid bilayer membranes dramatically increases membrane conductance, whereas the application of not-fibrillogenic rat IAPP has no effect on conductance (Mirzabekov et al. J Biol Chem. 1996, 271:1988-1992). Increases in membrane conductance (e.g:, influx of Ca⁺² and Na⁺ and efflux of K⁺) inevitably leads to cytotoxicity. Independent studies have demonstrated that human IAPP induces apoptosis in rat RINm5F cells (Zhang et al. FEBS Lett. 1999, 455:315-320; Saafi et al. Cell Biol lnt. 2001, 25:339-350). Membrane blebbing and microvilli loss were the earliest detectable apoptosisrelated phenomena, evident as early as 1 hour after HIAPP exposure. Following 6 to 12 hours of human IAPP-treatment, chromatin margination became evident, consistent with detection of DNA laddering at the same time. Nuclear shrinkage, nuclear membrane convolution and prominent cytoplasmic vacuolization were clearly recognized at 22 hours post-treatment.

Thus the development of anti-amyloidosis agents that are capable of preventing death of pancreatic β-cells remains an unmet medical need. Anti-amyloidosis agents for type 2 diabetes mellitus have been reported. α-Amino-γ-sulfonate and α-amino-δ-sulfonate derivatives are disclosed in U.S. Patent No. 6.562.836, while alky sulfate and sulfonate derivatives are disclosed in U.S. Patent Nos. 5,972,328 and 5,728,375. Bis- and tris-dihydroxyaryl compounds and their methylenedioxy analogs are disclosed in PCT Patent Application WO 031101927 A1. Glucose pentasulfate is disclosed in U.S. Patent No. 8,037,327. Derivatives of 1,2,3,4-tetrahydrolsochinoline are disclosed in PCT Patent Application WO 00/71101 A2. It was shown that anti-amyloidosis agents such as Congo Red (Lorenzo et al. Proc Natl Acad Sci. 1994, 91:12243-12247) and the peptide SNNFGA (Scrocchi et al. J Mol Biol. 2002, 318:697-706) completely or partially prevented β-cell death induced by a fibrillogenic form of IAPP. The main disadvantages with respect to the disclosed compounds are: a) potency was not reported (U.S. Patent No. 6,562,836, PCT Patent Application No. WO 00/71101 A2) or was low, between 1 and 20 mM (U.S. Patent Application Nos. 5,972,328, 6,037.327); b) no report of the effect of compounds on β-cells; and c) the effectiveness of compounds *in vivo* has not been described for any of the previously disclosed compounds.

Accordingly, there is a need for compounds and compositions to treat or prevent T2DM.

As disclosed in certain embodiments of the present invention, compounds of formulas I inhibit amyloidosis, prevent death of pancreatic β-cells and thus may be useful for the preparation of a medicament for treating or preventing T2DM. In certain embodiments, pharmaceutical compositions for the preparation of a medicament for the treatment or prevention of type 2 diabetes mellitus (T2DM), pathological consequences of T2DM, or inhibition of IAPP-Induced amyloidosis, or in the prevention of death of pancreatic β-cells comprise a pharmaceutical carrier, diluent or excipient and a compound of formula. Compounds of formulas I include the following: 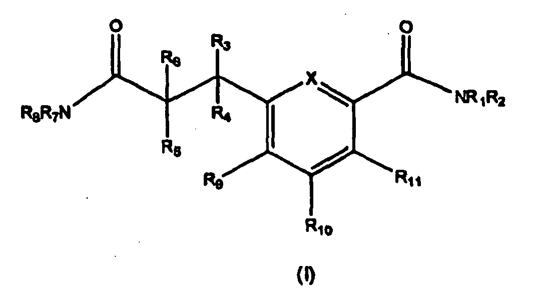 wherein X is C-H fragment or nitrogen;
R₁, R₂, R₇. R₈ are Independently selected from hydrogen and C₁-C₃ alkyl;
R₃, R₄, R₅, R₆ are independently selected from hydrogen, methyl, ethyl and propyl;
R₉, R₁₀, R₁₁, are independently selected from bromine, chlorine, fluorine, carboxy, hydrogen, hydroxyl, hydroxymethyl, methanesulfonamido, nitro, sulfamyl, trifluoromethyl, C₂₋C₇alkanoyloxy, C₁-C₆ alkyl, C₁-C₆ alkoxy, C₂-C₇ alkoxycarbonyl, C₁-C₆ thioalkyl, aryl, CON(R₃₈R₃₉) and N(R₃₈R₃₉) wherein R₃₈ and R₃₉ are independently selected from hydrogen, acetyl, methanesulfonyl and C₁-C₆ alkyl;
with the proviso that aromatic carbon atoms may be optionally replaced by aromatic nitrogen atoms.

In certain embodiments, pharmaceutical compositions for use in the preparation of a medicament for the treatment or prevention of type 2 diabetes mellitus (T2DM), pathological consequences of T2DM, or inhibition of IAPP-induced amyloidosis, or in the prevention of death of pancreatic β-cells comprise a pharmaceutical carrier, diluent or excipient and a compound of formula la or Ib:

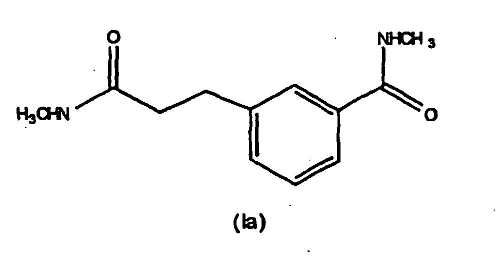

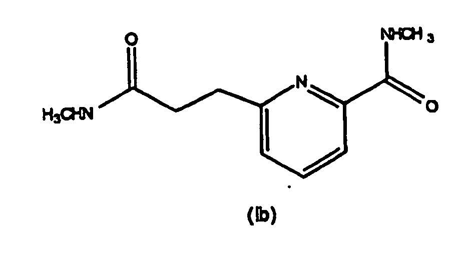

In certain embodiments, the present invention includes compound I: 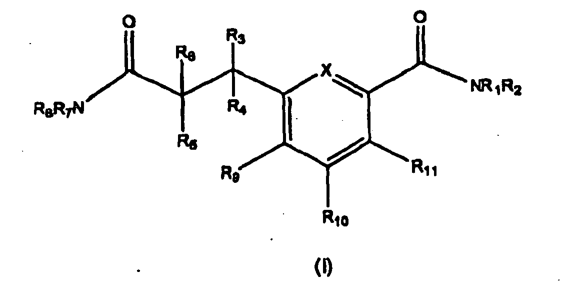 wherein X is C-H fragment or nitrogen;
R₁, R₂, R₇, R₈ are independently selected from hydrogen and C₁-C₃ alkyl;
R₃, R₄, R₅, R₆ are independently selected from hydrogen, methyl, ethyl and propyl;
R₈, R₁₀, R₁₁ are independently selected from bromine, chlorine, fluorine, carboxy, hydrogen, hydroxyl, hydroxymethyl, methanesulfonamido, nitro, sulfamyl, bifluoromethyl, C₂₋C₇ alkanoyloxy, C₁-C₈ alkyl, C₁-C₆ alkoxy, C_{2-C7} alkoxycarbonyl, C₁-C₈ thioalkyl, aryl, CON(R₃₈R₃₈) and
N(R₃₈R₃₉) wherein R₃₈ and R₃₉ are independently selected from hydrogen, acetyl, methanesulfonyl and C₁-C₈ alkyl; and
with the proviso that aromatic carbon atoms may be optionally replaced by aromatic nitrogen atoms.

In certain other embodiments, the present invention provides compounds I, Ia, Ib or compositions comprising compounds I, Ia or lb for use in the preparation of a medicament for the treatment or prevention of T2DM, pathological consequences of T2DM, or IAPP-induced amyloidosis, or prevention of death of pancreatic β-cells. In certain embodiments, the compound or composition may further include a pharmaceutical carrier, diluent or excipient

Compounds of formulas I, Ia, and Ib may be used in free or solvated form or as a pharmaceutically acceptable salt thereof and Include Isolated enantiomeric, diastereomeric and geometric isomers thereof, metabolites, metabolic precursors or prodrugs in crystalline, or amorphous, or liquid or gel forms including all polymorphic modifications thereof.

These and other aspects of the present invention will become apparent upon reference to the following detailed description.

As used herein, the following terms are defined as follows:
"Alkyl" refers to a branched or unbranched hydrocarbon fragment containing the specified number of carbon atoms and having one point of attachment. Examples include n-propyl (a C₃ alkyl), isopropyt (also a C₃ alkyl) and t-butyl (a C₄ alkyl).
"Alkoxyalkyl" refers to an alkylene group substituted with an alkoxy group. For example methyloxyethyl (CH₃OCH₂CH₃-) and ethoxymethyl (CH₃CH₂OCH₂-) are both C₃ alkoxyalkyl groups.
"Alkanoyloxy refers to an ester substituent wherein the ether oxygen is the point of attachment to the molecule. Examples include propanoyloxy (CH₃CH₂C(O)-O-), a C₃ alkanoyloxy and ethanoyloxy (CH₃C(O)-O-), a C₂ alkanoyloxy.
"Alkow" refers to an O-atom substituted by an alkyl group, for example methoxy (-OCH₃) a C₁ alkoxy.
"Alkoxycarbonyl" refers to an ester substituent wherein the carbonyl group is the point of attachment to the molecule. Examples Include ethoxycarbonyl (CH₃CH₂OC(O)-), a C₃ alkoxycarbonyl and methoxycarbonyl (CH₃OC(O)-), a C₂ alkoxycarbonyl.
"Aryl" refers to aromatic groups which have at least one ring having a conjugated pi electron system and includes carbocyclic aryl, heterocyclic aryl (also known as heteroaryl groups) and biaryl groups, all of which may be optionally substituted.
"Thioalkyl" refers to a sulfur atom substituted by an alkyl group, for example thiomethyl (CH₃S-), a C₁ thioalkyl.

Certain compounds of the present invention or for use in the pharmaceutical compositions of the present invention are represented by formula I: 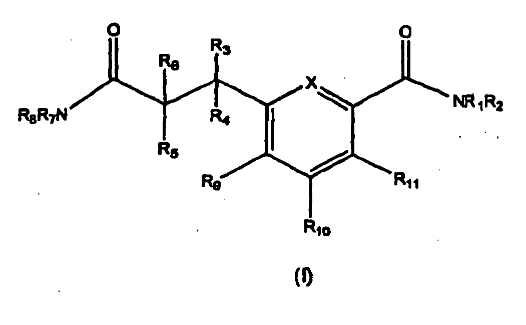 wherein X is C-H fragment or nitrogen;
R₁, R₂, R₇, R₈ are independently selected from hydrogen and C₁-C₃ alkyl;
R₃, R₄, R₅, R₆ are independently selected from hydrogen, methyl, ethyl and propyl;
R₉, R₁₀, R₁₁ are independently selected from bromine, chlorine, fluorine, carboxy, hydrogen, hydroxyl, hydroxymethyl, methanesulfonamido, nitro, sulfamyl, trifluoromethyl, C₂₋C₇ alkanoyloxy, C₁-C₈ alkyl, C₁-C₈ alkoxy, C₂-C₇ alkoxycarbonyl, C₁-C₈ thioalkyl, aryl, CON(R₃₈R₃₉) and N(R₃₈R₃₉) wherein R₃₈ and R₃₉ are Independently selected from hydrogen, acetyl, methanesulfonyl and C₁-C₆ alkyl;
with the proviso that aromatic carbon atoms may be optionally replaced by aromatic nitrogen atoms.

Compounds of formula I may be used in free or solvate form or in pharmaceutically acceptable salt thereof and include isolated enantiomeric, diastereomeric and geometric isomers thereof, metabolites, metabolic precursors or prodrugs In crystalline, or amorphous, or liquid or gel forms including all polymorphic modifications thereof.

One preferred embodiment of the present invention is a compound of formula la with the following structure:

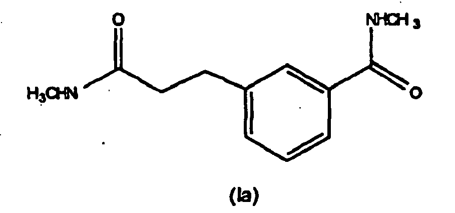

Another preferred embodiment of the present invention is a compound of formula Ib with the following structure:

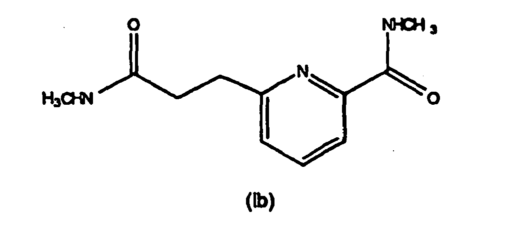

As disclosed in certain embodiments of the present invention, compounds of formula I may be useful for the preparation of a medicament for treating and/or preventing T2DM and pathological consequences of T2DM in warm-blooded animals, Including humans.

In certain embodiments the present invention provides compounds of formula I to inhibit IAPP-induced amyloidosis.

In further embodiments the present invention provides compounds of formula I to prevent death of pancreatic β-cell

The magnitude of the therapeutic or prophylactic dose of the compounds of the present invention in the treatment or prevention of T2DM, pathological consequences of T2DM, inhibition of amyloidosis and prevention of pancreatic β-cell death depends upon severity and nature of the condition being treated and the route of administration. The dose and the frequency of the dosing will also vary according to age, body weight and response of the individual patient. In general the total daily dose range for a compound of the present invention is from approximately 0.1 to approximately 500 mg in single or repeated doses.

Any suitable routes of administration may be employed to provide an effective dosage of the compounds of the present invention. Possible routes are not limited by oral, intravenous, topical and parenteral administrations, with oral administration representing a preferred route.

Compounds of the present invention may be administered in association with one or more inert carriers, excipients and diluents forming a pharmaceutical composition. Certain preferred oral compositions contain between approximately 0.1 % and approximately 75% of compounds of formulas I .

Solid compositions for oral administration may include binders, such as syrups, acacia, sorbitol, polyvinylpyrrolidone, carboxymethylcellulose, ethyl cellulose, microcrystalline cellulose or gelatin and mixtures thereof: excipients, such as starch, lactose or dextrins; disintegrating agents, such as alginic acid, sodium alginate, primogel and the like; lubricant, such as magnesium stearate, heavy molecular weight acids such as stearic acid, high molecular weight polymers such as polyethylene glycol; sweetening agents, such as sucrose or saccharine; flavoring agents, such as peppermint, methyl salicylate or orange flavoring; and coloring agents.

The liquid pharmaceutical compositions of the Invention, whether they are solutions, suspensions or other like form, may Include sterile diluents such as water for Injection, saline solution, preferably physiological saline, Ringer's solution, or isotonic sodium chloride, fixed oils such as synthetic mono or diglycerides which may serve as the solvent or suspending medium, polyethylene glycols, glycerin, propylene glycol or other solvents.

### EXAMPLES

### EXAMPLE 1

### SYNTHESES OF 3-(N-METHYLAMIDO)-N-METHYL PHENYL PROPANAMIDE (Ia)

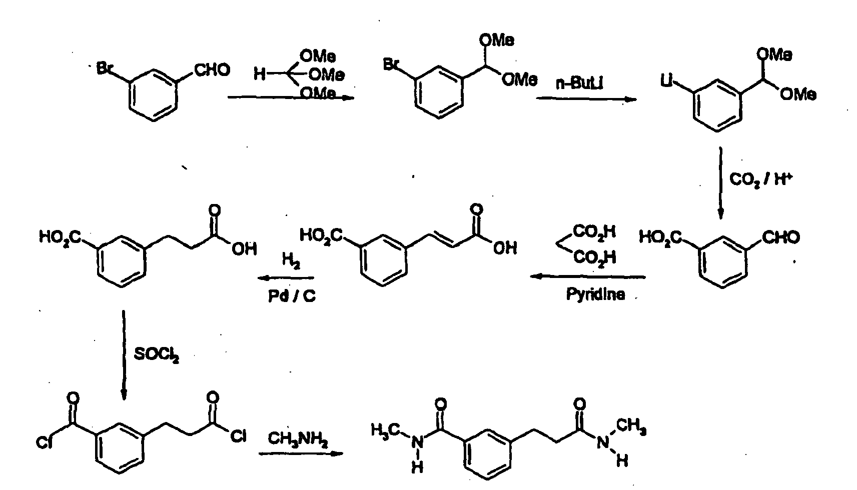

### Step-1:

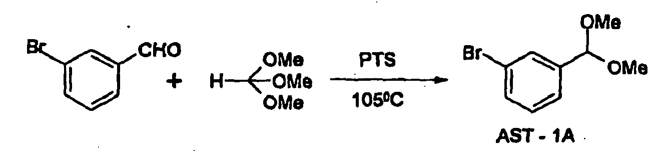

10 gm 3-Bromobenzaldehyde is taken in a 100ml round bottomed flask. To this is added 20 ml trimethyl orthoformate and 100 mg p-toluene sulphonic acid. Reaction mixture is then refluxed for 2 hours. TLC shows formation of the product. Heating is stopped, and the reaction mixture is extracted with hexane (3 x 500 ml), washed with sodium bicarbonate solution (5%) to remove the traces of p-toluenesulphonic acid. Reaction mixture is then dried over sodium sulphate and concentrated to give 12 gm dimethyl acetal of 3-bromobenzaldhyde (AST-1A).

### Step-2:

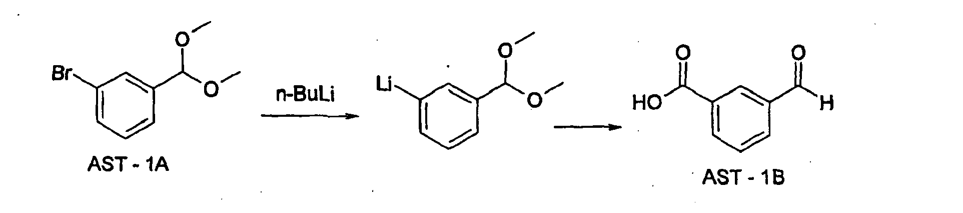

5 gm AST-1A is taken in 10 ml THF in a 3-necked round-bottomed flask and kept under nitrogen atmosphere in a tub containing dry ice to maintain the temperature at about -60°C. It is then stirred for 40 minutes. 3 gm dry ice is taken in a beaker and the reaction mixture is poured on dry ice with stirring. After complete addition, cold water and dilute hydrochloric acid are added to reaction mixture to a pH of 4. Then it is extracted with ethyl acetate (2x500 ml), dried over sodium sulfate and concentrated to give solid product (1.4 gm). Washing with hexane to remove impurities shown by PMR gives a final yield of 1.1 gm AST-1B.

### Step-3

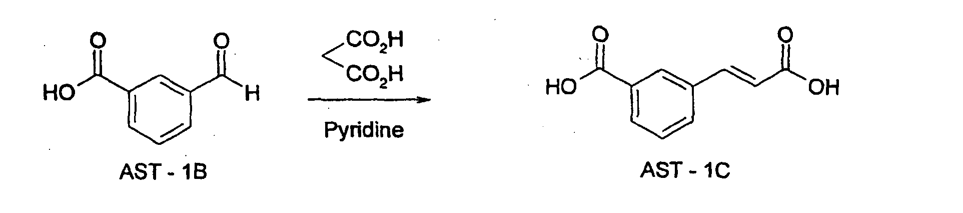

Malonic acid (1gm) and pyridine (3ml) is taken in a 100 ml RBF. It is kept in cold under stirring. To this is added 1 gm AST-1 B (3-formyl benzoic acid). The reaction mixture is stirred overnight at room temperature. Then it is heated for 1 hour, cooled and quenched in water, and extracted with ethyl acetate (2 x 500ml). The ethyl acetate layer is washed with dilute hydrochloric acid to pH 4.The ethyl acetate layer is then dried over sodium sulphate and concentrated to yield the product (1gm).

### Step-4

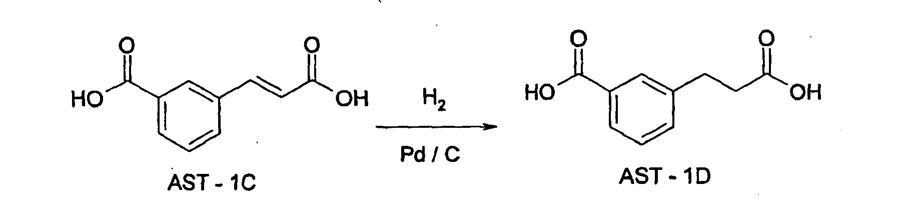

10% Palladium on charcoal (500mg) is added to 50 ml methanol in a round-bottomed flask and connected to a hydrogen cylinder through a trap. To this round bottomed flask is added 4 gm AST-1C and hydrogen gas is bubbled overnight. Stirring and hydrogen bubbling is stopped. The reaction mixture is filtered through Celite. The filtrate is collected, and methanol is removed by distillation under vacuum to give solid product (4 gm) AST-1 D. Reduction of the unsaturated compound is confirmed by NMR.

### Step-5

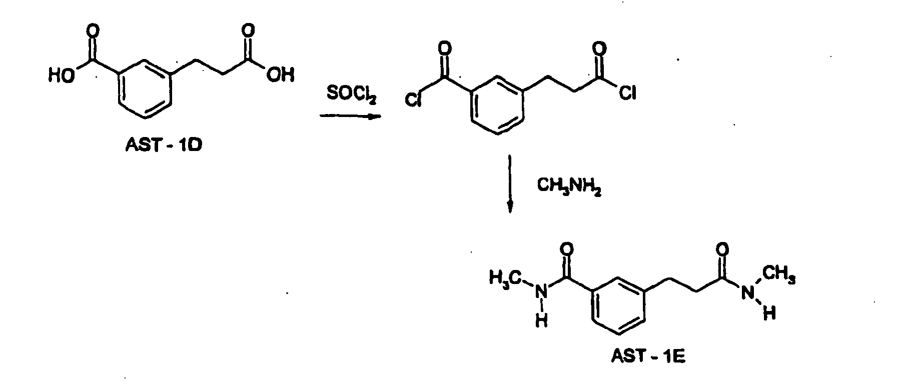

1gm AST-1D is placed in a 3-necked round-bottomed flask set with condenser and magnetic stirrer. To this is added 5 ml thionyl chloride, and the material is refluxed for 1 hour. Formation of acid chloride is confirmed by derivatizing it to ester and checking TLC. Excess thionyl chloride is then removed by distillation. 10 ml methylamine (liquefied) is placed In another round-bottomed flask maintained at -8°C. It is then stirred for half an hour and allowed to attain room temperature. The solid formed is filtered off, and the product obtained in the filtrate is concentrated and subjected to column chromatography using DCM and methanol to isolate 0.4 gm AST-1E ≡ la (final product).

### EXAMPLE 2.

### AMYLOID BINDING ASSAY

All test articles and control drugs are added (final concentrations 1-100 µM) to the wells of a 96-well plate (10 mM phenol red free Tris-HCl, pH 7.4) and incubated for 30 minutes at ca 37°C in humidified 5% CO₂ atmosphere, followed by the addition of cytotoxic target (IAPP) (final concentration 25 µM) to the appropriate wells. Thioflavin T (final concentration 5µM) is then added to the appropriate wells immediately following the addition of CTT. The plate is mixed gently on a gyratory shaker, Incubated at ca 37°C in a humidified 5% CO₂ atmosphere and read directly on a Tecan Satire reader in the fluorescence mode at excitation 450 nm and emission at 482 nm at 0,1, 3, and 6 hours. Experiments are run In duplicate on each of two plates.

### EXAMPLE 3

### CELL CULTURE AND CYTOTOXICITY ASSAYS

RINm5F cells are cultured in RPMI 1640 medium containing 10% fetal bovine serum, 290 µg/ml I-glutamine, 100 units/ml penicillin and 100 µg/ml streptomycin (Aitken et al. 2003). Cells are plated in 24-well plates at a density of 15 x 10⁴ cells per well, incubated for 48h, rinsed with PBS and placed in fresh medium (200 µl/well) in the presence or absence of compound I (final concentrations 0.1 1,10 and 100 µM). Following 30 minutes incubation, 12 µl of a freshly prepared aqueous solution of human IAPP (500 µM) is added to the cell culture medium to give final human IAPP concentration of 28 µM. Following 22h, cell viability is determined by double staining with calcein-AM and EthD-1. Green fluorescence of live cells and red fluorescence marking nuclei of dead cells are simultaneously visualized using a Zeiss axiovert S100 microscope equipped with a Zeiss filter set#09. Photographs are taken at 400x magnification using a Zeiss AxioCam digital camera. The EC₅₀ for the inhibition of cytotoxicity of IAPP by compounds I ranges from 0.6 µM to 100 µM.

## Claims

1. A pharmaceutical composition for the treatment or prevention of type 2 diabetes mellitus (T2DM), pathological consequences of T2DM, or inhibition of IAPP-induced amyloidosis, or in the prevention of death of pancreatic β-cells, comprising a pharmaceutical carrier, diluent or excipient and a compound of formula I: 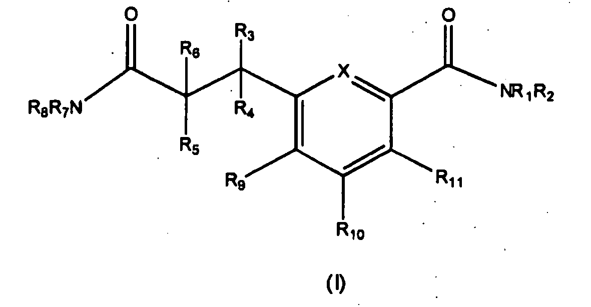 wherein X is C-H fragment or nitrogen;
R₁, R₂, R₇, R₈ are independently selected from hydrogen and C₁-C₃ alkyl;
R₃, R₄, R₅, R₆ are independently selected from hydrogen, methyl, ethyl and propyl;
R₉, R_{10,} R₁₁ are independently selected from bromine, chlorine, fluorine, carboxy, hydrogen, hydroxyl, hydroxymethyl_{;} methanesulfonamido, nitro, sulfamyl, trifluoromethyl, C₂-C₇ alkanoyloxy, C₁-C₆ alkyl, C₁-C₆ alkoxy, C₂-C₇ alkoxycarbonyl, C₁-C₆ thioalkyl, aryl, CON(R₃₈R₃₉) and N(R₃₈R₃₉) wherein R₃₈ and R₃₉ are independently selected from hydrogen, acetyl, methanesulfonyl and C₁-C₆ alkyl; and
with the proviso that aromatic carbon atoms may be optionally replaced by aromatic nitrogen atoms.

2. The pharmaceutical composition according to claim 1 for the treatment or prevention of T2DM, pathological consequences of T2DM, or inhibition of IAPP-induced amyloidosis, or in the prevention of death of pancreatic β-cells, comprising a pharmaceutical carrier, diluent or excipient and a compound of formula Ia or Ib:

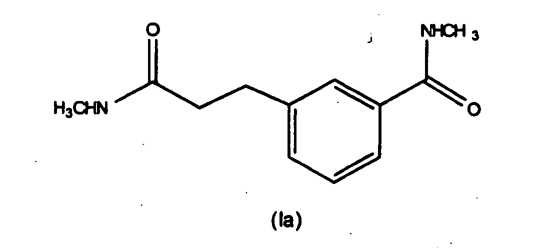

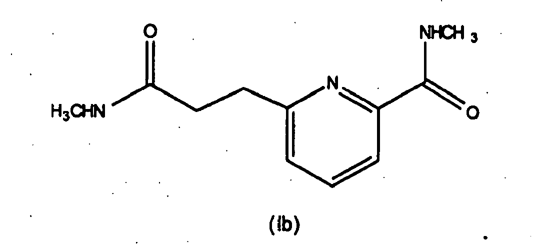

3. Use of a pharmaceutical composition for the preparation of a medicament for the treatment or prevention of type 2 diabetes mellitus (T2DM), pathological consequences of T2DM, or inhibition of IAPP-induced amyloidosis, or in the prevention of death of pancreatic β-cells, comprising a pharmaceutical carrier, diluent or excipient and a compound of formula I: 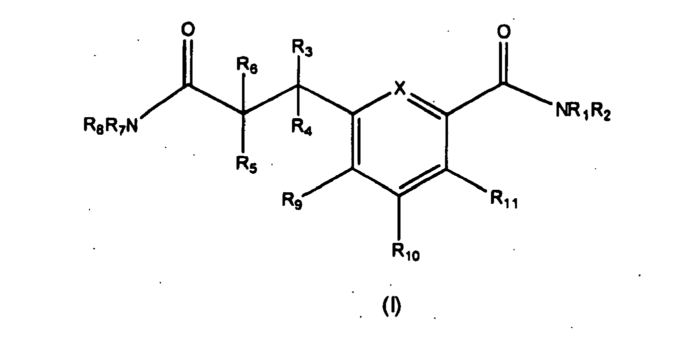 wherein X is C-H fragment or nitrogen;
R₁ R₂, R₇, R₈ are independently selected from hydrogen and C₁-C₃ alkyl;
R₃, R₄, R₅, R₆ are independently selected from hydrogen, methyl, ethyl and propyl;
R₉, R₁₀, R₁₁ are independently selected from bromine, chlorine, fluorine, carboxy, hydrogen, hydroxyl, hydroxymethyl, methanesulfonamido, nitro, sulfamyl, trifluoromethyl, C₂-C₇ alkanoyloxy, C₁-C₆ alkyl, C₁-C₆ alkoxy, C₂-C₇ alkoxycarbonyl, C₁-C₆ thioalkyl, aryl, CON(R₃₈R₃₉) and N(R₃₈R₃₉) wherein R₃₈ and R₃₉ are independently selected from hydrogen, acetyl, methanesulfonyl and C₁-C₆ alkyl; and
with the proviso that aromatic carbon atoms may be optionally replaced by aromatic nitrogen atoms.

4. Use of the pharmaceutical composition according to claim 3 for the preparation of a medicament for the treatment or prevention of T2DM, pathological consequences of T2DM, or inhibition of IAPP-induced amyloidosis, or in the prevention of death of pancreatic β-cells, comprising a pharmaceutical carrier, diluent or excipient and a compound of formula la or Ib:

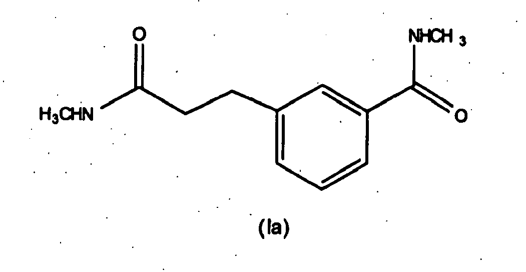

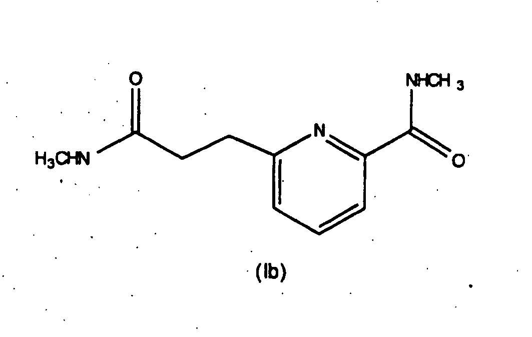

5. The composition according to claim 1 or 2 wherein the composition is for the treatment or prevention of T2DM or pathological consequences of T2DM.

6. Use of the composition according to claim 3 or 4 wherein the medicament is for the treatment or prevention of T2DM or pathological consequences of T2DM.

7. The composition according to claim 1 or 2 wherein the composition is for the treatment or prevention of IAPP-induced amyloidosis.

8. Use of the composition according to claim 3 or 4 wherein the medicament is for the treatment or prevention of IAPP-induced amyloidosis.

9. The composition according to claim 1 or 2 wherein the composition is for the prevention of death of pancreatic β-cells.

10. Use of the composition according to claim 3 or 4 wherein the medicament is for the prevention of death of pancreatic β-cells.

11. The use according to claim 6, 8, or 10 wherein the medicament includes a pharmaceutical carrier, diluent or excipient.

12. A compound of the formula I: 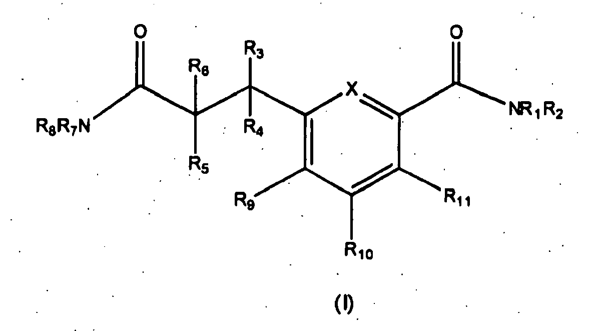 wherein X is C-H fragment or nitrogen;
R₁, R₂, R₇, R₈ are independently selected from hydrogen and C₁-C₃ alkyl;
R₃, R₄, R₅, R₆ are independently selected from hydrogen, methyl, ethyl and propyl;
R₉, R₁₀, R₁₁ are independently selected from bromine, chlorine, fluorine, carboxy, hydrogen, hydroxyl, hydroxymethyl, methanesulfonamido, nitro, sulfamyl, trifluoromethyl, C₂-C₇ alkanoyloxy, C₁-C₆ alkyl, C₁-C₆ alkoxy, C₂-C₇ alkoxycarbonyl, C₁-C₆ thioalkyl, aryl, CON(R₃₈R₃₉) and N(R₃₈R₃₈) wherein R₃₈ and R₃₉ are independently selected from hydrogen, acetyl, methanesulfonyl and C₁-C₆ alkyl; and
with the proviso that aromatic carbon atoms may be optionally replaced by aromatic nitrogen atoms.

13. The pharmaceutical composition according to claim 1 wherein
R₁₁ is selected from bromine, chlorine, fluorine, carboxy, hydrogen, methanesulfonamido, nitro, sulfamyl, trifluoromethyl, C₂-C₇ alkanoyloxy, C₁-C₆ alkyl, C₂-C₇ alkoxycarbonyl, C₁-C₆ thioalkyl, aryl, CON(R₃₈R₃₉) and N(R₃₈R₃₉) wherein R₃₈ and R₃₉ are independently selected from hydrogen, acetyl, methanesulfonyl and C₁-C₆ alkyl.

14. The compound according to claim 12 wherein
R₁₁ is selected from bromine, chlorine, fluorine, carboxy, hydrogen, methanesulfonamido, nitro, sulfamyl, trifluoromethyl, C₂-C₇ alkanoyloxy, C₁-C₆ alkyl, C₂-C₇ alkoxycarbonyl, C₁-C₆ thioalkyl, aryl, CON(R₃₈R₃₉) and N(R₃₈R₃₉) wherein R₃₈ and R₃₉ are independently selected from hydrogen, acetyl, methanesulfonyl and C₁-C₆ alkyl.

## Patentansprüche

1. Pharmazeutische Zusammensetzung für die Behandlung oder Verhütung von Typ 2 Diabetes mellitus (T2DM), pathologischen Folgen von T2DM, oder die Hemmung von IAPP-induzierter Amyloidose, oder die Verhütung des Todes von pankreatischen β-Zellen, umfassend einen pharmazeutischen Träger, ein pharmazeutisches Verdünnungsmittel oder einen pharmazeutischen Exzipienten und eine Verbindung der Formel I: 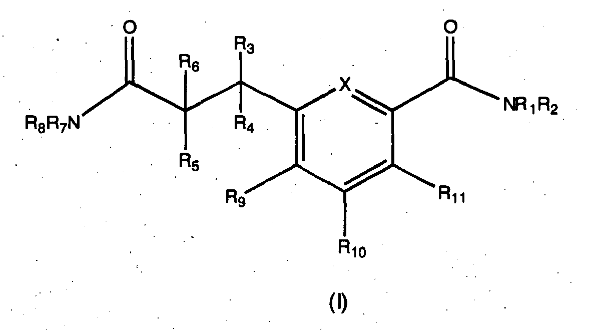 wobei X ein C-H Fragment oder Stickstoff ist;
R₁, R₂, R₇, R₈ unabhängig voneinander ausgewählt sind aus Wasserstoff und C₁-C₃-Alkyl;
R₃, R₄, R₅, R₆ unabhängig voneinander ausgewählt sind aus Wasserstoff, Methyl, Ethyl und Propyl;
R₉, R₁₀ R₁₁ unabhängig voneinander ausgewählt sind aus Brom, Chlor, Fluor, Carboxy, Wasserstoff, Hydroxyl, Hydroxymethyl, Methansulfonamido, Nitro, Sulfamyl, Trifluormethyl, C₂-C₇-Alkanoyloxy, C₁-C₆-Alkyl, C₁-C₆-Alkoxy, C₂-C₇-Alkoxycarbonyl, C₁-C₆-Thioalkyl, Aryl, CON(R₃₈R₃₉) und N(R₃₈R₃₉), wobei R₃₈ und R₃₉ unabhängig voneinander ausgewählt sind aus Wasserstoff, Acetyl, Methansulfonyl und C₁-C₆-Alkyl; und
mit der Maßgabe, dass aromatische Kohlenstoffatome gegebenenfalls durch aromatische Stickstoffatome ersetzt sein können.

2. Pharmazeutische Zusammensetzung nach Anspruch 1 für die Behandlung oder Verhütung von T2DM, pathologischen Folgen von T2DM, oder die Hemmung von IAPP-induzierter Amyloidose, oder die Verhütung des Todes von pankreatischen β-Zellen, umfassend einen pharmazeutischen Träger, ein pharmazeutisches Verdünnungsmittel oder einen pharmazeutischen Exzipienten und eine Verbindung der Formel la oder lb:

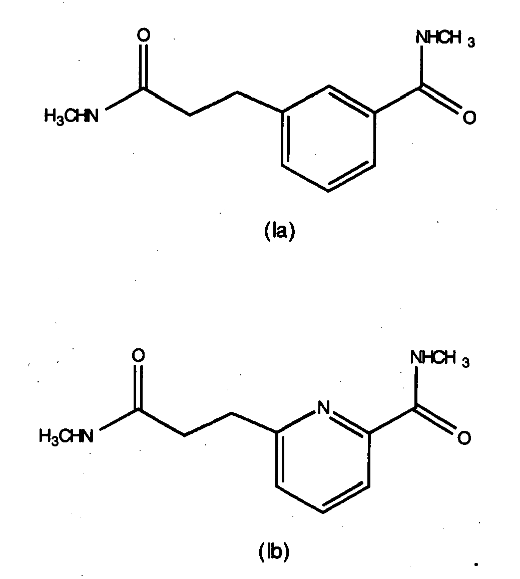

3. Verwendung einer pharmazeutischen Zusammensetzung für die Herstellung eines Medikaments für die Behandlung oder Verhütung von Typ 2 Diabetes mellitus (T2DM), pathologischen Folgen von T2DM, oder die Hemmung von IAPP-induzierter Amyloidose, oder die Verhütung des Todes von pankreatischen β-Zellen, umfassend einen pharmazeutischen Träger, ein pharmazeutisches Verdünnungsmittel oder einen pharmazeutischen Exzipienten und eine Verbindung der Formel 1: 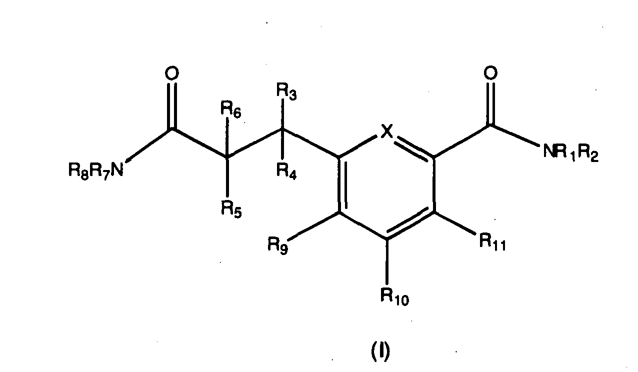 wobei X ein C-H Fragment oder Stickstoff ist;
R₁, R₂, R₇, R₈ unabhängig voneinander ausgewählt sind aus Wasserstoff und C₁-C₃-Alkyl;
R₃, R₄, R₅, R₆ unabhängig voneinander ausgewählt sind aus Wasserstoff, Methyl, Ethyl und Propyl;
R₉, R₁₀, R₁₁ unabhängig voneinander ausgewählt sind aus Brom, Chlor, Fluor, Carboxy, Wasserstoff, Hydroxyl, Hydroxymethyl, Methansulfonamido, Nitro, Sulfamyl, Trifluormethyl, C₂-C₇-Alkanoyloxy, C₁-C₆-Alkyl, C₁-C₆-Alkoxy, C₂-C₇-Alkoxycarbonyl, C₁-C₆-Thioalkyl, Aryl, CON(R₃₈R₃₉) und N(R₃₈R₃₉), wobei R₃₈ und R₃₉ unabhängig voneinander ausgewählt sind aus Wasserstoff, Acetyl, Methansulfonyl und C₁-C₆-Alkyl; und
mit der Maßgabe, dass aromatische Kohlenstoffatome gegebenenfalls durch aromatische Stickstoffatome ersetzt sein können.

4. Verwendung der pharmazeutischen Zusammensetzung nach Anspruch 3 für die Herstellung eines Medikaments für die Behandlung oder Verhütung von T2DM, pathologischen Folgen von T2DM, oder die Hemmung von IAPP-induzierter Amyloidose, oder die Verhütung des Todes von pankreatischen β-Zellen, umfassend einen pharmazeutischen Träger, ein pharmazeutisches Verdünnungsmittel oder einen pharmazeutischen Exzipienten und eine Verbindung der Formel la oder lb:

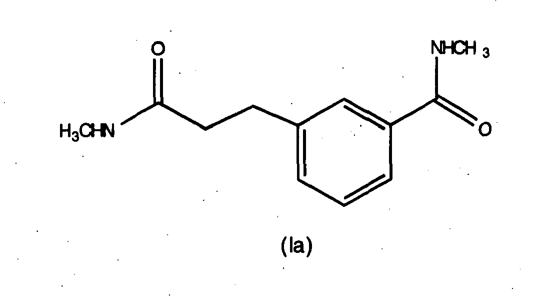

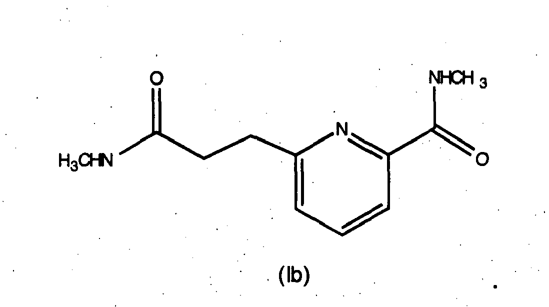

5. Zusammensetzung nach Anspruch 1 oder 2, wobei die Zusammensetzung für die Behandlung oder Verhütung von T2DM oder pathologischen Folgen von T2DM bestimmt ist.

6. Verwendung der Zusammensetzung nach Anspruch 3 oder 4, wobei das Medikament für die Behandlung oder Verhütung von T2DM oder pathologischen Folgen von T2DM bestimmt ist.

7. Zusammensetzung nach Anspruch 1 oder 2, wobei die Zusammensetzung für die Behandlung oder Verhütung von IAPP-induzierter Amyloidose bestimmt ist.

8. Verwendung der Zusammensetzung nach Anspruch 3 oder 4, wobei das Medikament für die Behandlung oder Verhütung von IAPP-induzierter Amyloidose bestimmt ist.

9. Zusammensetzung nach Anspruch 1 oder 2, wobei die Zusammensetzung für die Verhütung des Todes von pankreatischen β-Zellen bestimmt ist.

10. Verwendung der Zusammensetzung nach Anspruch 3 oder 4, wobei das Medikament für die Verhütung des Todes von pankreatischen β-Zellen bestimmt ist.

11. Verwendung nach Anspruch 6, 8 oder 10, wobei das Medikament einen pharmazeutischen Träger, ein pharmazeutisches Verdünnungsmittel oder einen pharmazeutischen Exzipienten einschließt.

12. Verbindung der Formel I: 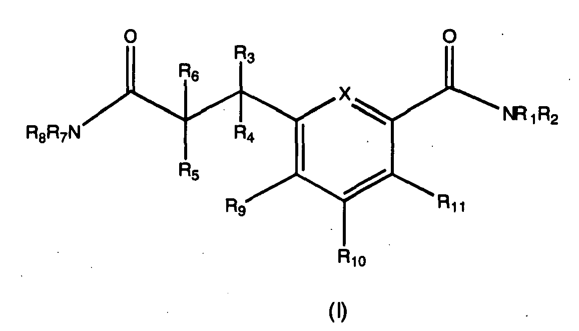 wobei X ein C-H Fragment oder Stickstoff ist;
R₁, R₂, R₇, R₈ unabhängig voneinander ausgewählt sind aus Wasserstoff und C₁-C₃-Alkyl;
R₃, R₄, R₅, R₆ unabhängig voneinander ausgewählt sind aus Wasserstoff, Methyl, Ethyl und Propyl;
R₉, R₁₀, R₁₁ unabhängig voneinander ausgewählt sind aus Brom, Chlor, Fluor, Carboxy, Wasserstoff, Hydroxyl, Hydroxymethyl, Methansulfonamido, Nitro, Sulfamyl, Trifluoromethyl, C₂-C₇-Alkanoyloxy, C₁-C₆-Alkyl, C₁-C₆-Alkoxy, C₂-C₇-Alkoxycarbonyl, C₁-C₆-Thioalkyl, Aryl, CON(R₃₈R₃₉) und N(R₃₈R₃₉), wobei R₃₈ und R₃₉ unabhängig voneinander ausgewählt sind aus Wasserstoff, Acetyl, Methansulfonyl und C₁-C₆-Alkyl; und
mit der Maßgabe, dass aromatische Kohlenstoffatome gegebenenfalls durch aromatische Stickstoffatome ersetzt sein können.

13. Pharmazeutische Zusammensetzung nach Anspruch 1, wobei R₁₁ ausgewählt ist aus Brom, Chlor, Fluor, Carboxy, Wasserstoff, Methansulfonamido, Nitro, Sulfamyl, Trifluormethyl, C₂-C₇-Alkanoyloxy, C₁-C₆-Alkyl, C₂-C₇-Alkoxycarbonyl, C₁-C₆-Thioalkyl, Aryl, CON(R₃₈R₃₉) und N(R₃₈R39), wobei R₃₈ und R₃₉ unabhängig voneinander ausgewählt sind aus Wasserstoff, Acetyl, Methansulfonyl und C₁-C₆-Alkyl.

14. Verbindung nach Anspruch 12, wobei R₁₁ ausgewählt ist aus Brom, Chlor, Fluor, Carboxy, Wasserstoff, Methansulfonamido, Nitro, Sulfamyl, Trifluormethyl, C₂-C₇-Alkanoyloxy, C₁-C₆-Alkyl, C₂-C₇-Alkoxycarbonyl, C₁-C₆-Thioalkyl, Aryl, CON(R₃₈R₃₉) und N(R₃₈R₃₉), wobei R₃₈ und R₃₉ unabhängig voneinander ausgewählt sind aus Wasserstoff, Acetyl, Methansulfonyl und C₁-C₆-Alkyl.

## Revendications

1. Composition pharmaceutique pour le traitement ou la prévention du diabète sucré de type 2 (T2DM), des conséquences pathologiques du T2DM, ou l'inhibition de l'amyloïdose induite par l'IAPP, ou la prévention de la mort de cellules β pancréatiques, comprenant un véhicule, diluant ou excipient pharmaceutique et un composé de formule I: 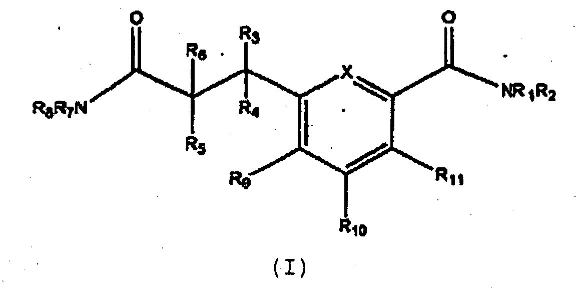 dans laquelle X est un fragment C-H ou l'azote ;
R₁, R₂, R₇ et R₈ sont indépendamment choisis parmi hydrogène et alkyle en C₁ à C₃ ;
R₃, R₄, R₅ et R₆ sont indépendamment choisis parmi hydrogène, méthyle, éthyle et propyle ;
R₉, R₁₀ et R₁₁ sont indépendamment choisis parmi brome, chlore, fluor, carboxy, hydrogène, hydroxyle, hydroxyméthyle, méthanesulfonamido, nitro, sulfamyle, trifluorométhyle, alcanoyloxy en C₂ à C₇, alkyle en C₁ à C₆, alcoxy en C₁ à C₆, alcoxycarbonyle en C₂ à C₇, thioalkyle en C₁ à C₆, aryle, CON (R₃₈R₃₉) et N (R₃₈R₃₉) où R₃₈ et R₃₉ sont indépendamment choisis parmi hydrogène, acétyle, méthanesulfonyle et alkyle en C₁ à C₆ ; et
avec la réserve que les atomes de carbone aromatiques peuvent être éventuellement remplacés par des atomes d'azote aromatiques.

2. Composition pharmaceutique selon la revendication 1 pour le traitement ou la prévention du T2DM, des conséquences pathologiques du T2DM, ou l'inhibition de l'amyloïdose induite par l'IAPP, ou la prévention de la mort de cellules β pancréatiques, comprenant un véhicule, diluant ou excipient pharmaceutique et un composé de formule Ia ou Ib :

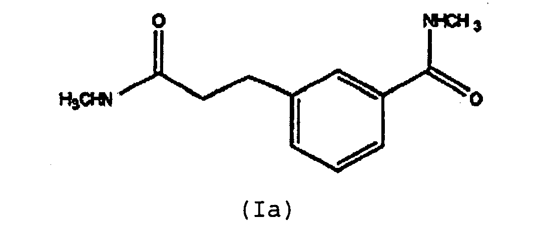

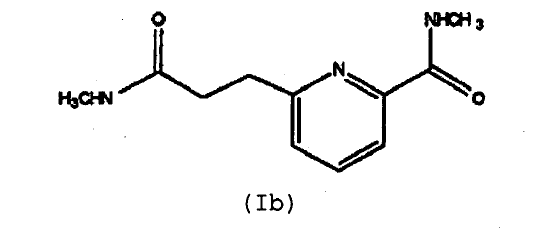

3. Utilisation d'une composition pharmaceutique pour la préparation d'un médicament pour le traitement ou la prévention du diabète sucré de type 2 (T2DM), des conséquences pathologiques du T2DM, ou l'inhibition de l'amyloïdose induite par l'IAPP, ou la prévention de la mort de cellules β pancréatiques, comprenant un véhicule, diluant ou excipient pharmaceutique et un composé de formule I: 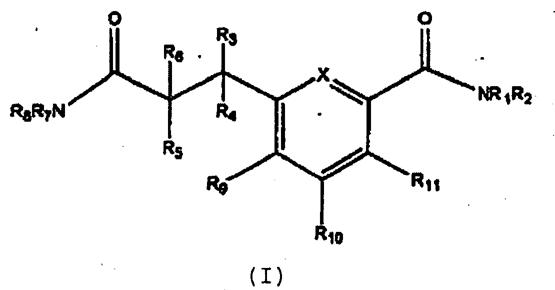 dans laquelle X est un fragment C-H ou l'azote ;
R₁, R₂, R₇ et R₈ sont indépendamment choisis parmi hydrogène et alkyle en C₁ à C₃;
R₃, R₄, R₅ et R₆ sont indépendamment choisis parmi hydrogène, méthyle, éthyle et propyle ;
R₉, R₁₀ et R₁₁ sont indépendamment choisis parmi brome, chlore, fluor, carboxy, hydrogène, hydroxyle, hydroxyméthyle, méthanesulfonamido, nitro, sulfamyle, trifluorométhyle, alcanoyloxy en C₂ à C₇, alkyle en C₁ à C₆, alcoxy en C₁ à C₆, alcoxycarbonyle en C₂ à C₇, thioalkyle en C₁ à C₆, aryle, CON (R₃₈R₃₉) et N (R₃₈R₃₉) où R₃₈ et R₃₉ sont indépendamment choisis parmi hydrogène, acétyle, méthanesulfonyle et alkyle en C₁ à C₆ ; et
avec la réserve que les atomes de carbone aromatiques peuvent être éventuellement remplacés par des atomes d'azote aromatiques.

4. Utilisation de la composition pharmaceutique selon la revendication 3 pour la préparation d'un médicament pour le traitement ou la prévention du T2DM, des conséquences pathologiques du T2DM, ou l'inhibition de l'amyloïdose induite par l'IAPP, ou la prévention de la mort de cellules β pancréatiques, comprenant un véhicule, diluant ou excipient pharmaceutique et un composé de formule Ia ou Ib :

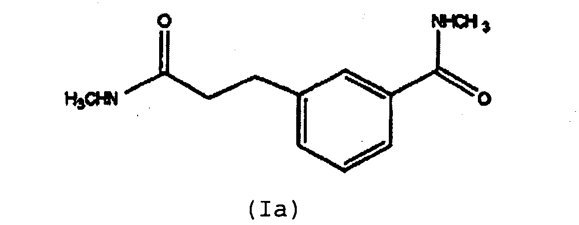

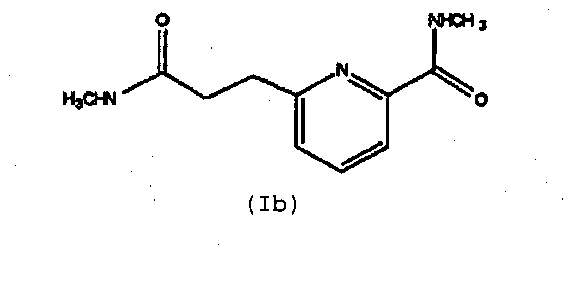

5. Composition selon la revendication 1 ou 2, qui est destinée au traitement ou à la prévention du T2DM ou des conséquences pathologiques du T2DM.

6. Utilisation de la composition selon la revendication 3 ou 4, dans laquelle le médicament est destiné au traitement ou à la prévention du T2DM ou des conséquences pathologiques du T2DM.

7. Composition selon la revendication 1 ou 2, qui est destinée au traitement ou à la prévention de l'amyloïdose induite par l'IAPP.

8. Utilisation de la composition selon la revendication 3 ou 4, dans laquelle le médicament est destiné au traitement ou à la prévention de l'amyloïdose induite par l'IAPP.

9. Composition selon la revendication 1 ou 2, qui est destinée à la prévention de la mort de cellules β pancréatiques.

10. Utilisation de la composition selon la revendication 3 ou 4, dans laquelle le médicament est destiné à la prévention de la mort de cellules β pancréatiques.

11. Utilisation selon la revendication 6, 8 ou 10, dans laquelle le médicament contient un véhicule, diluant ou excipient pharmaceutique.

12. Composé de formule I: 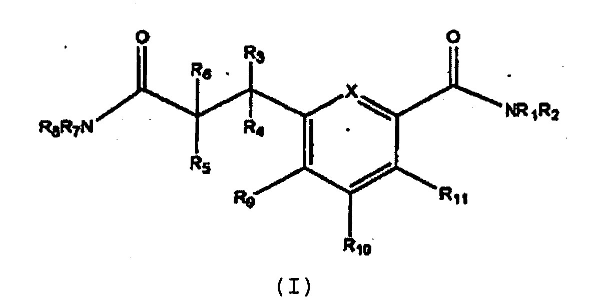 dans laquelle X est un fragment C-H ou l'azote ;
R₁, R₂, R₇ et R₈ sont indépendamment choisis parmi hydrogène et alkyle en C₁ à C₃ ;
R₃, R₄, R₅ et R₆ sont indépendamment choisis parmi hydrogène, méthyle, éthyle et propyle ;
R₉, R₁₀ et R₁₁ sont indépendamment choisis parmi brome, chlore, fluor, carboxy, hydrogène, hydroxyle, hydroxyméthyle, méthanesulfonamido, nitro, sulfamyle, trifluorométhyle, alcanoyloxy en C₂ à C₇, alkyle en C₁ à C₆, alcoxy en C₁ à C₆, alcoxycarbonyle en C₂ à C₇, thioalkyle en C₁ à C₆, aryle, CON (R₃₈R₃₉) et N (R₃₈R₃₉) où R₃₈ et R₃₉ sont indépendamment choisis parmi hydrogène, acétyle, méthanesulfonyle et alkyle en C₁ à C₆ ; et
avec la réserve que les atomes de carbone aromatiques peuvent être éventuellement remplacés par des atomes d'azote aromatiques.

13. Composition pharmaceutique selon la revendication 1 dans laquelle R₁₁ est choisi parmi brome, chlore, fluor, carboxy, hydrogène, méthanesulfonamido, nitro, sulfamyle, trifluorométhyle, alcanoyloxy en C₂ à C₇, alkyle en C₁ à C₆, alcoxycarbonyle en C₂ à C₇, thioalkyle en C₁ à C₆, aryle, CON (R₃₈R₃₉) et N (R₃₈R₃₉) où R₃₈ et R₃₉ sont indépendamment choisis parmi hydrogène, acétyle, méthanesulfonyle et alkyle en C₁ à C₆;

14. Composé selon la revendication 12 dans lequel R₁₁ est choisi parmi brome, chlore, fluor, carboxy, hydrogène, méthanesulfonamido, nitro, sulfamyle, trifluorométhyle, alcanoyloxy en C₂ à C₇, alkyle en C₁ à C₆, alcoxycarbonyle en C₂ à C₇, thioalkyle en C₁ à C₆, aryle, CON (R₃₈R₃₉) et N (R₃₈R₃₉) où R₃₈ et R₃₉ sont indépendamment choisis parmi hydrogène, acétyle, méthanesulfonyle et alkyle en C₁ à C₆.
